(19) Europäisches Patentamt
European Patent Office
Office européen des brevets

(11) **EP 4 449 443 B1**

(12) **EUROPEAN PATENT SPECIFICATION**

(45) Date of publication and mention
of the grant of the patent:
**26.11.2025 Bulletin 2025/48**

(21) Application number: **22823633.7**

(22) Date of filing: **12.12.2022**

(51) International Patent Classification (IPC):
*G16H 50/50* (2018.01)   *G16H 50/20* (2018.01)
*G06T 17/00* (2006.01)   *A61B 5/00* (2006.01)
*A61B 5/026* (2006.01)   *A61B 6/03* (2006.01)
*A61B 6/50* (2024.01)   *A61B 6/00* (2024.01)

(52) Cooperative Patent Classification (CPC):
**G16H 50/50; G06T 17/00; G16H 50/20;**
A61B 5/0044; A61B 5/026; A61B 6/032;
A61B 6/504; A61B 6/5217; G06T 2210/41

(86) International application number:
**PCT/IB2022/062070**

(87) International publication number:
**WO 2023/111814 (22.06.2023 Gazette 2023/25)**

(54) **COMPUTER-IMPLEMENTED METHOD FOR THE SIMULATION OF MYOCARDIAL BLOOD FLOW UNDER STRESS CONDITIONS**

COMPUTERIMPLEMENTIERTES VERFAHREN ZUR SIMULATION DES MYOKARDIALEN BLUTFLUSSES UNTER STRESSBEDINGUNGEN

PROCÉDÉ MIS EN OEUVRE PAR ORDINATEUR POUR LA SIMULATION D'UN DÉBIT SANGUIN MYOCARDIQUE DANS DES CONDITIONS DE STRESS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.12.2021 IT 202100031475**

(43) Date of publication of application:
**23.10.2024 Bulletin 2024/43**

(73) Proprietor: **Centro Cardiologico Monzino SpA**
**20121 Milano (MI) (IT)**

(72) Inventors:
• **DI GREGORIO, Simone**
**20133 Milano (MI) (IT)**
• **PONTONE, Gianluca**
**20121 Milano (MI) (IT)**
• **QUARTERONI, Alfio**
**20133 Milano (MI) (IT)**

• **VERGARA, Christian**
**20133 Milano (MI) (IT)**

(74) Representative: **Grana, Daniele**
**BRUNACCI & PARTNERS S.r.l.**
**Via Pietro Giardini, 625**
**41125 Modena (MO) (IT)**

(56) References cited:
**US-A1- 2021 334 963**

• **DI GREGORIO SIMONE ET AL: "A computational model applied to myocardial perfusion in the human heart: From large coronaries to microvasculature", JOURNAL OF COMPUTATIONAL PHYSICS, LONDON, GB, vol. 424, 18 September 2020 (2020-09-18), XP086317410, ISSN: 0021-9991, [retrieved on 20200918], DOI: 10.1016/J.JCP.2020.109836**

EP 4 449 443 B1

**Description**

<u>Technical field of the invention</u>

The present invention relates to a computer-implemented method for the simulation of myocardial blood flow under stress conditions.

<u>State of the art</u>

The myocardial perfusion, also known as myocardial blood flow (MBF), is the delivery of blood to the heart muscle, named myocardium, supplied by the coronary circulation.
The quantification of MBF and the functional assessment of coronary artery disease (CAD) could be achieved through stress myocardial computed tomography perfusion (stress-CTP).
This technique requires an additional scan after the coronary computed tomography angiography at rest (cCTA) and an intravenous stressor administration, leading to an increase of radiation exposure for the patient and potential stressor's related side effects.
Computational methods could reveal an effective tool as a concrete support for clinicians, allowing a completely noninvasive diagnostic technique for myocardial perfusion quantification and for coronary stenosis detection.
To this purpose, in document "A computational model applied to myocardial perfusion in the human heart: From large coronaries to microvasculature." (Journal of Computational Physics, 424:109836, 2021), a multi-physics mathematical and numerical model of myocardial perfusion was proposed to quantify MBF avoiding the stress protocol and the related potential side effects and reducing the radiation exposure.
However, the mathematical and numerical model per se is not sufficient to quantify MBF in stress conditions, indeed it requires a suitable set of patient-specific parameters, which can allow the numerical simulations to compute MBF in stress conditions.
Document US 2021/334963 A1 further discloses methods and systems provided for assessing the presence of functionally significant stenosis in one or more coronary arteries.

<u>Summary of the invention</u>

Therefore, the main aim of the present invention is to provide a computer-implemented method for the simulation of myocardial blood flow under stress conditions that allows to compute MBF in stress conditions in an effective and accurate way for the specific patient.
The above-mentioned objects are achieved by the present computer-implemented method for the simulation of myocardial blood flow under stress conditions according to the features of claim 1.

<u>Description of the figures</u>

Other characteristics and advantages of the present invention will become better evident from the description of a preferred, but not exclusive embodiments of a computer-implemented method for the simulation of myocardial blood flow under stress conditions, illustrated by way of an indicative but non-limiting example in the accompanying Figures, in which:

Figure 1 is a block diagram of the computer-implemented method according to the invention;
Figure 2 schematically shows vasodilation under stress conditions simulated for a physical parameters adjustment step of the computer-implemented method according to the invention.

<u>Detailed description of preferred embodiments</u>

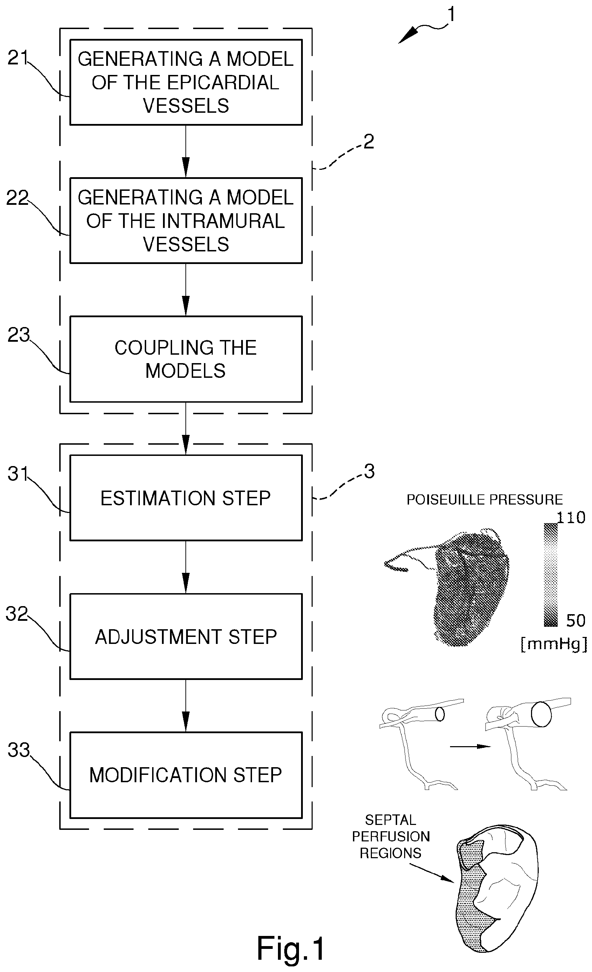

With particular reference to figure 1, globally indicated with reference 1 is a computer-implemented method for the simulation of myocardial blood flow under stress conditions.
The computer-implemented method 1 according to the invention comprises a first step 2 of generating a simulated multi-physics model of a myocardial perfusion.
Particularly, in the coronary artery tree a clear scale separation can be observed between the main vessels laying on the epicardium, the epicardial vessels, and the smaller vessels penetrating into the tissue, the intramural vessels (The multi-scale modelling of coronary blood flow. Annals of Biomedical Engineering, 40(11):2399-2413, 2012).
Therefore, because of such scale separation, the step 2 of generating the simulated multi-physics model of a myocardial perfusion comprises:

- a step 21 of generating a simulated model of the epicardial vessels by means of a three-dimensional fluid-dynamics description;
- a step 22 of generating a simulated model of the intramural vessels by means of a multi-compartment porous medium.

According to a preferred embodiment, the step of generating a simulated model of the epicardial vessels using a three-dimensional fluid-dynamics description is implemented using incompressible Navier-Stokes equations.

Furthermore, according to the preferred embodiment, the step of generating a simulated model of the intramural vessels by means of a multi-compartment porous medium is implemented using Darcy's law (Multi-scale parameterisation of a myocardial perfusion model using whole-organ arterial networks. Annals of Biomedical Engineering, 42(4):797-811, 2014).

Furthermore, the step 2 of generating the multi-physics simulated model of a myocardial perfusion comprises a step 23 of coupling the simulated model of the epicardial vessels and the simulated model of the intramural vessels using interface conditions based on the continuity of mass and momentum in every perfusion regions, wherein each perfusion region is a specific myocardial territory perfused by a distinct epicardial vessel (Visualisation of intramural coronary vasculature by an imaging cryomicrotome suggests compartmentalization of myocardial perfusion areas. Medical and Biological Engineering and Computing, 43(4):431-435, 2005).

Particularly, in case of three compartments, the step 2 of generating a multi-physics simulated model of a myocardial perfusion can be implemented by executing the following expressions:

$$\rho\left(\frac{\partial u_C}{\partial t} + (u_C \cdot \nabla)u_C\right) - \mu\nabla \cdot \left(\nabla u_C + (\nabla u_C)^T\right) + \nabla p_C = \mathbf{0}$$

in $\Omega_C$.

$$\nabla \cdot u_C = 0$$

in $\Omega_C$,

$$p_C - \mu\left(\nabla u_C + (\nabla u_C)^T\right)n \cdot n - \frac{1}{\omega^j}\int_{\Gamma^j} u_C \cdot n\, d\gamma = \frac{1}{|\Omega_M^j|}\int_{\Omega_M^j} p_{M,1}\, dx$$

on $\Gamma^j$,

$$\mu(\nabla u_C + (\nabla u_C)^T)n \cdot \tau_i = 0 \qquad i = 1, 2$$

on $\Gamma^j$,

$$u_{M,1} + K_1\nabla p_{M,1} = \mathbf{0}$$

in $\Omega_M$.

$$\nabla \cdot u_{M,1} = \sum_{j=1}^J \frac{\chi_{\Omega_M^j}}{|\Omega_M^j|}\int_{\Gamma^j} u_C \cdot n\, d\gamma - \beta_{1,2}(p_{M,1} - p_{M,2})$$

in $\Omega_M$,

$$u_{M,2} + K_2\nabla p_{M,2} = \mathbf{0}$$

in $\Omega_M$,

$$\nabla \cdot u_{M,2} = -\beta_{2,1}(p_{M,2} - p_{M,1}) - \beta_{2,3}(p_{M,2} - p_{M,3})$$

in $\Omega_M$,

$$u_{M,3} + K_3 \nabla p_{M,3} = 0$$

in $\Omega_M$,

$$\nabla \cdot u_{M,3} = -\gamma(p_{M,3} - p_{veins}) - \beta_{3,2}(p_{M,3} - p_{M,2})$$

in $\Omega_M$, wherein:

$\Omega_C$ is the domain of the epicardial coronary arteries;
$\Omega_M$ is the domain of the intramural vessels;
$\Omega^J_M$, j = 1, ..., J, are the domains of the perfusion regions;
uc and pc are the blood velocity and pressure, respectively, in the epicardial coronary arteries;
$\rho$ is the blood density;
$\mu$ is the blood viscosity;
n is the unit outer normal vector;
$\alpha^j$ are the conductances between the epicardial coronary arteries and the intramural vessels.

Furthermore, for i = 1 ... 3 in the i-th compartment:

$u_{M,i}$ and $p_{M,i}$ are the Darcy velocity and the pore pressure, respectively;
$K_i$ is the permeability tensor;
$\beta_{i,k} \geq 0$, i, k = 1 ...N, represent the inter-compartment pressure-coupling coefficients between the compartments i and k;
$p_{veins}$ is the venous pressure;
$\gamma$ is a suitable drain coefficient.

Finally, the notation $\chi_A$ stands for the characteristic function of the domain A.
Particularly, to enforce mass conservation among compartments, we have that $\beta_{i,k} = \beta_{k,i}$, Vi , k = 1, ... , N.
Moreover, $\beta_{i,k} \neq 0$ whenever $k = i \pm 1$ for $2 \leq i \leq N - 1$, $k = 2$ for $i = 1$, $k = N - 1$ for $i = N$, since the compartment $i$ exchanges mass only with adjacent compartments. Particularly, only the first compartment is involved in the coupling condition and the average of pressure on the whole compartment $\Omega j$ is considered due to the homogenized nature of the Darcy equation.
Particularly, it is pointed out that the step 2 of generating the multi-physics simulated model can be implemented as disclosed in the document "A computational model applied to myocardial perfusion in the human heart: From large coronaries to microvasculature (Journal of Computational Physics, 424:109836, 2021).
To reproduce clinical data of a myocardial blood flow (MBF) maps, the simulation implemented by the computer-implemented method 1 according to the invention require a proper set of specific physical parameters of the simulated multi-physics model of a myocardial perfusion.
Advantageously, the computer-implemented method 1 according to the invention comprises a step 3 of automatic calibration of physical parameters of the multi-physics simulated model of a myocardial perfusion under stress conditions.
Particularly, said calibrated physical parameters are:

the permeability tensors $K_i$, i = 1, 2, 3;
the conductances $\alpha^j$, j = 1, ... , 1, between the epicardial coronary arteries and the intramural vessels; and
the inter-compartment conductances $\beta_{i,k}$, i, k = 1, 2, 3, between the compartments i and k.

Suitable values of such parameters are estimated for each patient and assumed to vary among the different perfusion regions.
As schematically showed in Figure 1, the step 3 of automatic calibration of the physical parameters under stress conditions comprises the following steps:

- an estimation step 31 of the physical parameters in rest conditions, by exploiting the intramural vessels geometrical and fluid dynamics properties in rest conditions;
- an adjustment step 32 of the physical parameters accounting vasodilation under stress conditions;
- a modification step 33 of the physical parameters at the septum, particularly by increasing of physical parameters in the septum.

According to the estimation step 31, the intramural vascular network is exploited to estimate first possible values for the physical parameters in rest conditions.

As for the permeability tensors $K_i$, $i = 1, 2, 3$, they are initialized based on geometric issues, in particular for each compartment and perfusion region they were given by the ratio between the volume of the intramural vessels in such region and the total region volume.

The first step consists in the separation of the intramural vascular network into two groups of vessels using a specific metric. This is motivated by the fact that it is possible to relate the largest vessels to the first compartment, whereas the smallest ones to the second compartment.

Particularly, it is pointed out that the surrogate intramural vascular network generated according to the document "A computational model applied to myocardial perfusion in the human heart: From large coronaries to microvasculature" (Journal of Computational Physics, 424:109836, 2021) does not include the microvasculature, which is the part of the network modelled in the third Darcy compartment.

To perform such operation, the estimation step 31 comprises a definition step of a hierarchic parameter $\zeta \in [0, 1]$ for each node $y_i$ of the intramural vascular network.

Particularly, the definition step comprises calculating the hierarchic parameter $\zeta(y_i)$ as the ratio between the sum of the lengths of the vessels which are located distally to $y_i$ and the sum of the lengths of all the vessels of the network.

In this way $\zeta$ will be 1 for the most proximal nodes and 0 for the most distal terminal nodes.

Then, given for each perfusion region $\Omega^j_M$ a value $Z^j \in (0, 1)$, the estimation step 31 comprises a sorting step for sorting a vessel of the network to belong to the first or second group of vessels if the average of the values of $\zeta$ in the nodes of the vascular network at hand is in the range $[0, Z^j]$ or in the range $[Z^j, 1]$.

The values $Z^j$ are chosen in order to have about the same number of vessels in the two groups.

Particularly, the estimation step 31 comprises calculating the global constant permeability tensor $K_i$ as:

$$K_i(\mathbf{x}) = \sum_{j=1}^{J} K_i^j \chi_{\Omega^j_M}(\mathbf{x})$$

where $K_i^j$ is the permeability tensor of the *i-th* compartment in the perfusion region $\Omega^j_M$.

Particularly, the permeability tensor $K_i^j$ is assumed isotropic and is defined as:

$$K_i^j = \phi_i^j I$$

where $I$ is the identity tensor with unit of $cm^2 Pa^{-1} s^{-1}$ and $\Phi_i^j$ is the constant porosity.

Particularly, the constant porosity $\Phi_i^j$ is defined as follows:

$$\phi_i^j = \frac{\sum_{n=1}^{M_i^j} V_{i,n}^j}{V_{\Omega^j_M}};$$

where $V_{\Omega^j_M}$ is the volume of $\Omega^j_M$, $M_i^j$ is the number of the vessels in $\Omega^j_M$, and $V_{i,n}^j$ is the volume of the *n-th* vessel in the *i-th* compartment of $\Omega^j_M$.

To compute the conductances $\beta_{i,k}$ and $\alpha^j$, other information about the pressure and flow distributions in the vascular network are required. Particularly, in order to find an approximation of the conductances $\beta_{i,k}$ and $\alpha^j$, the solution of a Poiseuille flow problem along vascular network is considered, given by the union between the epicardial coronaries and the intramural network.

To reduce the computational effort, the epicardial coronaries are modeled as 1D tubular structures (notice that this is done only for the parameter estimation, whereas elsewhere in the model the coronaries are 3D).

As for the boundary conditions, an inlet pressure of 109 *mmHg* and outlet pressures depending on the radius of the terminal vessels are prescribed.

A constant multiplicative correction factor $\eta$ is in case applied to all the vessels radii in order to obtain a physiological flow rate starting from this pressure gradient.

Referring to this Poiseuille solution, $Q_{i,k}^j = Q_{k,i}^j$, $i, k = 1, 2, 3$, is the total flow rate exchanged in the perfusion region $\Omega^j_M$ at the interface between compartment *i* and compartment *k*.

Since there are not any vessels in the third compartment, $Q_{2,3}^j$ is set to be equal to the total flow rate at the outlet of the second compartment of $\Omega^j_M$.

Moreover, $p^j_{i,n}$, $i = 1, 2$, is the pressure in the *n-th* vessel of compartment *i* in the perfusion region $\Omega^j_M$ and

$$\bar{p}^j_3 = 39\text{mmHg}$$ a reference value for the microvasculature pressure for all j, computed as the average value between the pressure of the most downstream vessels ($\approx 56$ *mmHg*) and the value of $p_{veins}$ = 22.5 *mmHg*.

Particularly, the estimation step 31 comprises calculating the global piecewise constant inter-compartment conductance $\beta_{i,k}$ as:

$$\beta_{i,k}(\mathbf{x}) = \sum_{j=1}^{J} \beta^j_{i,k} \chi_{\Omega^j_M}(\mathbf{x})$$

where $\beta^j_{i,k}$ is a local coupling coefficient.

Particularly, the local coupling coefficient $\beta^j_{i,k}$ inside the perfusion region $\Omega^j_M$ is defined as:

$$\beta^j_{1,2} = \begin{cases} 0 & \text{if } \bar{p}^j_1 - \bar{p}^j_2 = 0, \\ \dfrac{\overline{Q}^j_{1,2}}{|\bar{p}^j_1 - \bar{p}^j_2|} & \text{otherwise,} \end{cases}$$

$$\beta^j_{2,3} = \begin{cases} 0 & \text{if } \bar{p}^j_2 - \bar{p}^j_3 = 0, \\ \dfrac{\overline{Q}^j_{2,3}}{|\bar{p}^j_2 - \bar{p}^j_3|} & \text{otherwise,} \end{cases}$$

$$\beta^j_{i,k} = 0 \quad \text{elsewhere,}$$

where

$$\overline{Q}^j_{i,k} = \frac{Q^j_{i,k}}{V_{\Omega^j_M}}$$

and

$$\bar{p}^j_i = \frac{\sum_{n=1}^{M^j_i} p^j_{i,n} V^j_{i,n}}{\sum_{n=1}^{M^j_i} V^j_{i,n}} \quad i = 1, 2$$

In a similar way, the estimation step 31 comprises calculating the conductance coefficient $\alpha^j$ as:

$$\alpha^j = \frac{Q^j_{inlet}}{|p^j_{inlet} - \bar{p}^j_1|}, \quad j = 1, \dots, J$$

where $Q^j_{inlet}$ is the flow rate entering in the first compartment of $\Omega^j_M$ and $p^j_{inlet}$ is the pressure in the first node of the first compartment of $\Omega^j_M$.

After computing the physical parameters under rest conditions, the stressor agent effect on the coronary arteries shall be accounted. To this purpose the epicardial coronary artery domain $\Omega_C$, which was reconstructed from rest CT images, was post-processed to account for the vasodilation.

Particularly, the adjustment step 32 of the physical parameters comprises executing the following steps:

- choosing a sample epicardial coronary artery, which is visible on axial scans acquired under rest and stress conditions;
- measuring the value of the radius under rest conditions $R_{rest-saple}$ and the value of the radius under stress conditions $R_{stress-sample}$ in the sample epicardial coronary artery;
- compute the vasodilation factor $v_{str}$ as

$$v_{str} = \frac{R_{stress-sample}}{R_{rest-sample}}$$

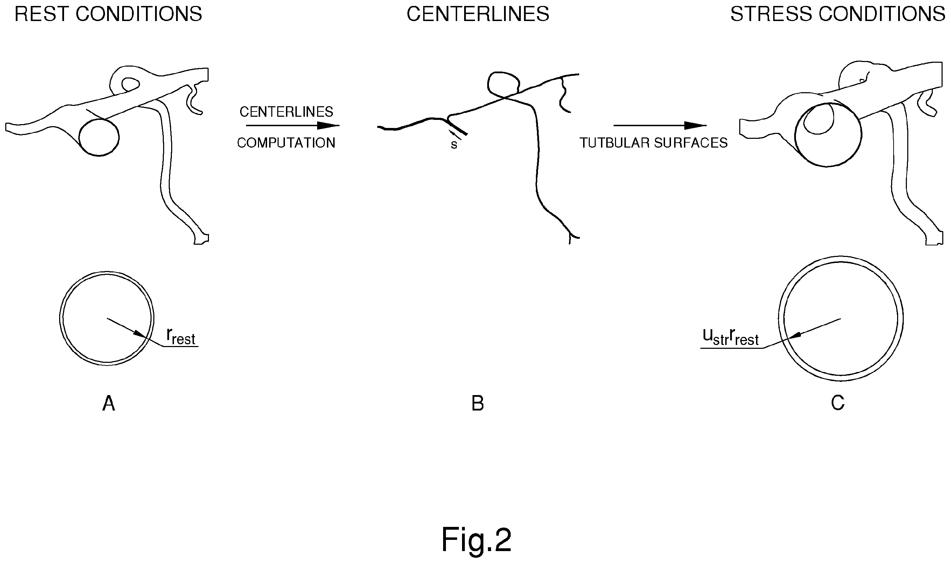

- compute the centerlines of the epicardial coronary arteries reconstructed from rest computed tomography angiography (CTA) and compute the radius of the vessels in each point of the centerlines (Figure 2 from A to B);
- generate a new epicardial coronary arteries surface by extruding a tubular surface from the centerlines (Figure 2 from B to C), whose radius in each tract is computed as

$$r_{stress}(s) = v_{str}\, r_{rest}(s)$$

where s is the curvilinear abscissa along the centerlines.

Moreover, according to physiological evidences, the adenosine injection leads to an increase of the vascular resistance of about 10-fold. For this reason, in order to account for the vasodilation in the intramural vascular network, the physical resistive parameters of the multi-compartment Darcy model $\beta_{1,2}$ and $\beta_{2,3}$, $K_1$ and $K_2$ are increased by 10-fold with respect to baseline parameters estimated for resting conditions at said estimation step.

A final adjustment is performed based on the observation that at the septum the values of $\beta_{1,2}$ and $\beta_{2,3}$ estimated at said adjustment step are lower than the other myocardial regions, leading to a systematic underestimation of MBF computed by the numerical simulations with respect to MBF estimated with stress-CTP.

To overcome this, the modification step 33 comprises multiplying the inter-compartment pressure-coupling coefficients $\beta_{1,2}$ and $\beta_{2,3}$ by a factor 5 in perfusion regions located at the ventricular septum.

The present invention is also related to an apparatus for coronary computed tomography angiography at rest (cCTA) configured for executing the steps of the computer-implemented method 1 as disclosed above.

Therefore, the apparatus according to the invention comprises all the hardware and software conventionally needed for the coronary computed tomography angiography at rest (cCTA) and a further elaboration unit configured for executing he computer-implemented method 1.

## Claims

1. Computer-implemented method for the simulation of myocardial blood flow under stress conditions, executed on an apparatus for coronary computed tomography angiography at rest, comprising a step (2) of generating a simulated multi-physics model of a myocardial perfusion, wherein said step (2) of generating further comprises:

   - a step (21) of generating a simulated model of the epicardial vessels by means of a three-dimensional fluid-dynamics description;
   - a step (22) of generating a simulated model of the intramural vessels by means of a multi-compartment porous medium;
   - a step (23) of coupling the simulated model of the epicardial vessels and the simulated model of the intramural vessels;

   **characterized in that** it comprises a step (3) of automatic calibration of physical parameters of the simulated multi-physics model of a myocardial perfusion under stress conditions, wherein said calibrated physical parameters are:

   - permeability tensors ($K_i$, $i$ = 1, 2, 3);
   - conductances between the epicardial coronary arteries and the intramural vessels ($\alpha^j$, $j = 1, ... , J$); and
   - inter-compartment conductances ($\beta_{i,k}$, $i, k$ = 1, 2, 3) between the compartments ($i, k$);

wherein said step (3) of automatic calibration comprises the following steps:

- an estimation step (31) of the physical parameters in rest conditions, by exploiting the intramural vessels geometrical and fluid dynamics properties in rest conditions;
- an adjustment step (32) of the physical parameters accounting vasodilation under stress conditions;
- a modification step (33) of the physical parameters at the septum, particularly by increasing of physical parameters in the septum;

and wherein said adjustment step (32) of the physical parameters comprises executing the following steps:

- choosing a sample epicardial coronary artery, which is visible on axial scans acquired under rest and stress conditions;
- measuring the value of the radius under rest conditions $R_{rest-saple}$ and the value of the radius under stress conditions $R_{stress-sample}$ in the sample epicardial coronary artery;
- compute the vasodilation factor $v_{str}$ as

$$v_{str} = \frac{R_{stress-sample}}{R_{rest-sample}}$$

- compute the centerlines of the epicardial coronary arteries reconstructed from rest computed tomography angiography (CTA) and compute the radius of the vessels in each point of the centerlines;
- generate a new epicardial coronary arteries surface by extruding a tubular surface from the centerlines, whose radius in each tract is computed as

$$r_{stress}(s) = v_{str} \, r_{rest}(s)$$

where s is the curvilinear abscissa along the centerlines.

2. Computer-implemented method according to claim 1, **characterized in that** said estimation step (31) comprises calculating the global constant permeability tensor $K_i$ as:

$$K_i(\mathbf{x}) = \sum_{j=1}^{J} K_i^j \chi_{\Omega_M^j}(\mathbf{x})$$

where $K_i^j$ is the permeability tensor of the *i-th* compartment in a perfusion region $\Omega_M^j$.

3. Computer-implemented method according to claim 2, **characterized in that** said permeability tensor $K_i^j$ is defined as:

$$K_i^j = \phi_i^j I$$

where $I$ is the identity tensor with unit of $cm^2 Pa^{-1} s^{-1}$ and $\Phi_i^j$ is the constant porosity.

4. Computer-implemented method according to claim 3, **characterized in that** said constant porosity $\Phi_i^j$ is defined as follows:

$$\phi_i^j = \frac{\sum_{n=1}^{M_i^j} V_{i,n}^j}{V_{\Omega_M^j}}$$

where $V_{\Omega_M^j}$ is the volume of $\Omega_M^j$, $M_i^j$ is the number of the vessels in $\Omega_M^j$, and $V_{i,n}^j$ is the volume of the *n-th* vessel in the *i-th* compartment of $\Omega^j_M$.

**5.** Computer-implemented method according to one or more of the preceding claims, **characterized in that** said estimation step (31) comprises calculating the global piecewise constant inter-compartment conductances $\beta_{i,\,k}$ as:

$$\beta_{i,k}(\mathbf{x}) = \sum_{j=1}^{J} \beta^j_{i,k} \chi_{\Omega^j_M}(\mathbf{x})$$

where $\beta^j_{i,\,k}$ is a local coupling coefficient.

**6.** Computer-implemented method according to claim 5, **characterized in that** said local coupling coefficient $\beta^j_{i,\,k}$ inside the perfusion region $\Omega^j_M$ is defined as:

$$\beta^j_{1,2} = \begin{cases} 0 & \text{if } \overline{p}^j_1 - \overline{p}^j_2 = 0, \\ \dfrac{\overline{Q}^j_{1,2}}{\left|\overline{p}^j_1 - \overline{p}^j_2\right|} & \text{otherwise,} \end{cases}$$

$$\beta^j_{2,3} = \begin{cases} 0 & \text{if } \overline{p}^j_2 - \overline{p}^j_3 = 0, \\ \dfrac{\overline{Q}^j_{2,3}}{\left|\overline{p}^j_2 - \overline{p}^j_3\right|} & \text{otherwise,} \end{cases}$$

$$\beta^j_{i,k} = 0 \quad \text{elsewhere,}$$

where

$$\overline{Q}^j_{i,k} = \frac{Q^j_{i,k}}{V_{\Omega^j_M}}$$

and

$$\overline{p}^j_i = \frac{\sum_{n=1}^{M^j_i} p^j_{i,n} V^j_{i,n}}{\sum_{n=1}^{M^j_i} V^j_{i,n}} \quad i = 1, 2$$

**7.** Computer-implemented method according to one or more of the preceding claims, **characterized in that** said estimation step (31) comprises calculating the conductance coefficient $\alpha^j$ as:

$$\alpha^j = \frac{Q^j_{inlet}}{\left|p^j_{inlet} - \overline{p}^j_1\right|}, \quad j = 1, \ldots, J$$

where $Q^j_{inlet}$ is the flow rate entering in the first compartment of $\Omega^j_M$ and $p^j_{inlet}$ is the pressure in the first node of the first compartment of $\Omega^j_M$.

8. Computer-implemented method according to one or more of the preceding claims, **characterized in that** said modification step (33) comprises multiplying the inter-compartment pressure-coupling coefficients ($\beta_{1,2}$, $\beta_{2,3}$) by a predefined factor in the septal perfusion regions.

9. Apparatus for coronary computed tomography angiography at rest (cCTA) configured for executing the steps of the computer-implemented method (1) for the simulation of myocardial blood flow under stress conditions according to one or more of the preceding claims.

**Patentansprüche**

1. Computerimplementiertes Verfahren zur Simulation des myokardialen Blutflusses unter Belastungsbedingungen, das auf einem Gerät zur koronaren Computertomographie-Angiographie im Ruhezustand ausgeführt wird, umfassend einen Schritt (2) zum Erzeugen eines simulierten multiphysikalischen Modells einer myokardialen Perfusion, wobei der Schritt (2) zum Erzeugen ferner umfasst:

   - einen Schritt (21) zum Erzeugen eines simulierten Modells der epikardialen Gefäße mittels einer dreidimensionalen fluid-dynamischen Beschreibung;
   - einen Schritt (22) zum Erzeugen eines simulierten Modells der intramuralen Gefäße mittels eines porösen Mediums mit mehreren Kompartimenten;
   - einen Schritt (23) zum Koppeln des simulierten Modells der epikardialen Gefäße und des simulierten Modells der intramuralen Gefäße;
   **dadurch gekennzeichnet, dass** es einen Schritt (3) der automatischen Kalibrierung physikalischer Parameter des simulierten multiphysikalischen Modells einer myokardialen Perfusion unter Stressbedingungen umfasst, wobei die kalibrierten physikalischen Parameter sind:

      - Permeabilitätstensoren ($K_i$, $i$ = 1, 2, 3);
      - Leitfähigkeiten zwischen den epikardialen Koronararterien und den intramuralen Gefäßen ($\alpha^j$, $j$ = 1, ... , $J$); und
      - Interkompartiment-Leitfähigkeiten ($\beta_{i,k}$, $i$, $k$= 1, 2, 3) zwischen den Kompartimenten ($i$, $k$);

   wobei der Schritt (3) der automatischen Kalibrierung die folgenden Schritte umfasst:

      - einen Schritt (31) zur Schätzung der physikalischen Parameter unter Ruhebedingungen unter Ausnutzung der geometrischen und fluidodynamischen Eigenschaften der intramuralen Gefäße unter Ruhebedingungen;
      - einen Schritt (32) zum Anpassen der physikalischen Parameter unter Berücksichtigung der Vasodilatation unter Stressbedingungen;
      - einen Schritt (33) zur Modifizierung der physikalischen Parameter am Septum, insbesondere durch Erhöhung der physikalischen Parameter in dem Septum;

   und wobei der Anpassungsschritt (32) der physikalischen Parameter die Ausführung der folgenden Schritte umfasst:

      - Auswahl einer Probe einer epikardialen Koronararterie, die auf axialen Scans sichtbar ist, die unter Ruhe- und Stressbedingungen aufgenommen wurden;
      - Messung des Radiuswerts unter Ruhebedingungen $R_{rest\text{-}saple}$ und des Radiuswerts unter Belastungsbedingungen $R_{stress\text{-}sample}$ in der Probe der epikardialen Koronararterie;
      - Berechnung des Vasodilatationsfaktors $v_{str}$ als

$$v_{str} = \frac{R_{stress-sample}}{R_{rest-sample}}$$

      - Berechnung der Mittellinien der epikardialen Koronararterien, die aus der Computertomographie-Angiographie (CTA) unter Ruhebedingungen rekonstruiert wurden, und Berechnung des Radius der Gefäße an jedem Punkt der Mittellinien;

- Erzeugen einer neuen Oberfläche der epikardialen Koronararterien durch Extrudieren einer röhrenförmigen Oberfläche aus den Mittellinien, deren Radius in jedem Abschnitt berechnet wird als

$$r_{stress}(s) = v_{str}\, r_{rest}(s)$$

wobei s die gekrümmte Abszisse entlang der Mittellinien ist.

2. Computerimplementiertes Verfahren nach Anspruch 1, **dadurch gekennzeichnet, dass** der Schätzungsschritt (31) das Berechnen des globalen konstanten Permeabilitätstensors $K_i$ umfasst als:

$$K_i(\mathbf{x}) = \sum_{j=1}^{J} K_i^j \chi_{\Omega_M^j}(\mathbf{x})$$

wobei $K_i^j$ der Permeabilitätstensor des *i-ten* Kompartiments in einer Perfusionsregion $\Omega_M^j$ ist.

3. Computerimplementiertes Verfahren nach Anspruch 2, **dadurch gekennzeichnet, dass** der Permeabilitätstensor $K_i^j$ definiert ist als:

$$K_i^j = \phi_i^j I$$

wobei $I$ der Identitätstensor mit der Einheit $cm^2 Pa^{-1} s^{-1}$ ist und $\Phi_i^j$ die konstante Porosität ist.

4. Computerimplementiertes Verfahren nach Anspruch 3, **dadurch gekennzeichnet, dass** die konstante Porosität $\Phi_i^j$ definiert ist als:

$$\phi_i^j = \frac{\sum_{n=1}^{M_i^j} V_{i,n}^j}{V_{\Omega_M^j}},$$

wobei $V_{\Omega_M^j}$ das Volumen von $\Omega_M^j$ ist, $M_i^j$ die Anzahl der Gefäße in $\Omega_M^j$ ist und $V_{i,n}^j$ das Volumen des *n-ten* Gefäßes im *i-ten* Kompartiment von $\Omega_M^j$ ist.

5. Computerimplementiertes Verfahren nach einem oder mehreren der vorhergehenden Ansprüche, **dadurch gekennzeichnet, dass** der Schätzungsschritt (31) ein Berechnen der globalen stückweise konstanten Zwischenkompartiment-Leitfähigkeiten $\beta_{i,k}$ umfasst als:

$$\beta_{i,k}(\mathbf{x}) = \sum_{j=1}^{J} \beta_{i,k}^j \chi_{\Omega_M^j}(\mathbf{x})$$

wobei $\beta_{i,k}^j$ ein lokaler Kopplungskoeffizient ist.

6. Computerimplementiertes Verfahren nach Anspruch 5, **dadurch gekennzeichnet, dass** der lokale Kopplungskoeffizient $\beta_{i,k}^j$ innerhalb des Perfusionsbereichs $\Omega_M^j$ definiert ist als:

$$\beta_{1,2}^j = \begin{cases} 0 & \text{if } \overline{p}_1^j - \overline{p}_2^j = 0, \\ \dfrac{\overline{Q}_{1,2}^j}{|\overline{p}_1^j - \overline{p}_2^j|} & \text{otherwise,} \end{cases}$$

(otherwise = sonst)

$$\beta_{2,3}^{j} = \begin{cases} 0 & \text{if } \overline{p}_2^j - \overline{p}_3^j = 0, \\ \dfrac{\overline{Q}_{2,3}^j}{|\overline{p}_2^j - \overline{p}_3^j|} & \text{otherwise,} \end{cases}$$

(otherwise = sonst)

$$\beta_{i,k}^{j} = 0 \quad \text{elsewhere,}$$

, (elsewhere = anderswo),

wobei

$$\overline{Q}_{i,k}^{j} = \frac{Q_{i,k}^j}{V_{\Omega_M^j}}$$

und

$$\overline{p}_i^j = \frac{\sum_{n=1}^{M_i^j} p_{i,n}^j V_{i,n}^j}{\sum_{n=1}^{M_i^j} V_{i,n}^j} \quad i = 1, 2$$

.

7. Computerimplementiertes Verfahren nach einem oder mehreren der vorhergehenden Ansprüche, **dadurch gekennzeichnet, dass** der Schätzungsschritt (31) das Berechnen des Leitfähigkeitskoeffizienten $\alpha^j$ umfasst als:

$$\alpha^j = \frac{Q_{inlet}^j}{|p_{inlet}^j - \overline{p}_1^j|}, \quad j = 1, \dots, J$$

wobei $Q_{inlet}^j$ die in das erste Kompartiment von $\Omega_M^j$ eintretende Flussrate ist und $p_{inlet}^j$ der Druck im ersten Knoten des ersten Kompartiments von $\Omega_M^j$ ist.

8. Computerimplementiertes Verfahren nach einem oder mehreren der vorhergehenden Ansprüche, **dadurch gekennzeichnet, dass** der Modifizierungsschritt (33) das Multiplizieren der Druckkopplungskoeffizienten ($\beta_{1,2}$, $\beta_{2,3}$) zwischen den Kompartimenten mit einem vordefinierten Faktor in den septalen Perfusionsbereichen umfasst.

9. Vorrichtung für die koronare Computertomographie-Angiographie in Ruhe (cCTA), die zur Ausführung der Schritte des computerimplementierten Verfahrens (1) zur Simulation des myokardialen Blutflusses unter Belastungsbedingungen nach einem oder mehreren der vorhergehenden Ansprüche konfiguriert ist.

**Revendications**

1. - Procédé mis en œuvre par ordinateur pour la simulation d'un débit sanguin myocardique dans des conditions de stress, exécuté sur un appareil pour angiographie coronarienne par tomographie assistée par ordinateur au repos, comprenant une étape (2) de génération d'un modèle multi-physique simulé d'une perfusion myocardique, ladite étape (2) de génération comprenant en outre :

   - une étape (21) de génération d'un modèle simulé des vaisseaux épicardiques au moyen d'une description de dynamique des fluides en trois dimensions;
   - une étape (22) de génération d'un modèle simulé des vaisseaux intramuraux au moyen d'un milieu poreux à plusieurs compartiments ;
   - une étape (23) de couplage du modèle simulé des vaisseaux épicardiques et du modèle simulé des vaisseaux intramuraux ;

**caractérisé par le fait qu'**il comprend une étape (3) de calibrage automatique de paramètres physiques du modèle multi-physique simulé d'une perfusion myocardique dans des conditions de stress, lesdits paramètres physiques calibrés étant :

- des tenseurs de perméabilité ($K_i$, $i$ = 1, 2, 3) ;
- des conductances entre les artères coronaires épicardiques et les vaisseaux intramuraux ($\alpha^j$, $j$ = 1, ..., $J$) ; et
- des conductances inter-compartiments ($\beta_{i,k}$, $i$, $k$ = 1, 2, 3) entre les compartiments ($i$, $k$) ;

dans lequel ladite étape (3) de calibrage automatique comprend les étapes suivantes :

- une étape d'estimation (31) des paramètres physiques dans des conditions de repos, par exploitation des propriétés géométriques et de dynamique des fluides des vaisseaux intramuraux dans des conditions de repos ;
- une étape d'ajustement (32) des paramètres physiques rendant compte de la vasodilatation en conditions de stress ;
- une étape de modification (33) des paramètres physiques au septum, en particulier par augmentation de paramètres physiques dans le septum ;

et dans lequel ladite étape d'ajustement (32) des paramètres physiques comprend l'exécution des étapes suivantes :

- choisir une artère coronaire épicardique d'échantillon, qui est visible sur des balayages axiaux acquis dans des conditions de repos et de stress ;
- mesurer la valeur du rayon dans des conditions de repos $R_{rest\text{-}sample}$ et la valeur du rayon dans des conditions de stress $R_{stress\text{-}sample}$ dans l'artère coronaire épicardique d'échantillon ;
- calculer le facteur de vasodilatation $v_{str}$ comme :

$$v_{str} = \frac{R_{stress-sample}}{R_{rest-sample}}$$

- calculer les lignes centrales des artères coronaires épicardiques reconstruites à partir d'une angiographie par tomographie assistée par ordinateur (CTA) au repos et calculer le rayon des vaisseaux en chaque point des lignes centrales ;
- générer une nouvelle surface des artères coronaires épicardiques par extrusion d'une surface tubulaire à partir des lignes centrales, dont le rayon dans chaque tractus est calculé comme :

$$r_{stress}(s) = v_{str}\, r_{rest}(s)$$

où $s$ est l'abscisse curviligne le long des lignes centrales.

2. - Procédé mis en œuvre par ordinateur selon la revendication 1, **caractérisé par le fait que** ladite étape d'estimation (31) comprend le calcul du tenseur de perméabilité constant global $K_i$ comme:

$$K_i(x) = \sum_{j=1}^{J} K_i^j \chi_{\Omega_M^j}(x)$$

où $K_i^j$ est le tenseur de perméabilité du $i$-*ième* compartiment dans une région de perfusion $\Omega_M^j$.

3. - Procédé mis en œuvre par ordinateur selon la revendication 2, **caractérisé par le fait que** ledit tenseur de perméabilité $K_i^j$ est défini comme :

$$K_i^j = \phi_i^j I$$

où $I$ est le tenseur d'identité avec l'unité de $cm^2 Pa^{-1} s^{-1}$ et $\phi^j i$ est la porosité constante.

4. - Procédé mis en œuvre par ordinateur selon la revendication 3, **caractérisé par le fait que** ladite porosité constante $\phi^j i$ est définie comme suit :

$$\phi_i^j = \frac{\sum_{n=1}^{M_i^j} V_{i,n}^j}{V_{\Omega_M^j}},$$

où $V\Omega_M^j$ est le volume de $\Omega_M^j$, $M_i^j$ est le nombre des vaisseaux dans $\Omega_M^j$, et $V_{i,n}^j$ est le volume du *n-ième* vaisseau dans le *i-ème* compartiment de $\Omega_M^j$.

5. - Procédé mis en œuvre par ordinateur selon une ou plusieurs des revendications précédentes, **caractérisé par le fait que** ladite étape d'estimation (31) comprend le calcul des conductances inter-compartiments constantes par morceaux globales $\beta_{i,k}$ comme :

$$\beta_{i,k}(\mathbf{x}) = \sum_{j=1}^{J} \beta_{i,k}^j \chi_{\Omega_M^j}(\mathbf{x})$$

où $\beta_{i,k}^j$ est un coefficient de couplage local.

6. - Procédé mis en œuvre par ordinateur selon la revendication 5, **caractérisé par le fait que** ledit coefficient de couplage local $\beta^j{}_{i,k}$ à l'intérieur de la région de perfusion $\Omega_M^j$ est défini comme :

$$\beta_{1,2}^j = \begin{cases} 0 & \text{si } \overline{p}_1^j - \overline{p}_2^j = 0, \\ \dfrac{\overline{Q}_{1,2}^j}{|\overline{p}_1^j - \overline{p}_2^j|} & \text{autrement,} \end{cases}$$

$$\beta_{2,3}^j = \begin{cases} 0 & \text{si } \overline{p}_2^j - \overline{p}_3^j = 0, \\ \dfrac{\overline{Q}_{2,3}^j}{|\overline{p}_2^j - \overline{p}_3^j|} & \text{autrement,} \end{cases}$$

$$\beta_{i,k}^j = 0 \quad \text{sinon,}$$

où

$$\overline{Q}_{i,k}^j = \frac{Q_{i,k}^j}{V_{\Omega_M^j}}$$

et

$$\overline{p}_i^j = \frac{\sum_{n=1}^{M_i^j} p_{i,n}^j V_{i,n}^j}{\sum_{n=1}^{M_i^j} V_{i,n}^j} \quad i = 1, 2$$

**7.** - Procédé mis en œuvre par ordinateur selon une ou plusieurs des revendications précédentes, **caractérisé par le fait que** ladite étape d'estimation (31) comprend le calcul du coefficient de conductance $\alpha^j$ comme :

$$\alpha^j = \frac{Q_{inlet}^j}{|p_{inlet}^j - \overline{p}_1^j|}, \quad j = 1, \dots, J$$

où $Q_{inlet}^j$ est le débit entrant dans le premier compartiment de $\Omega_M^j$ et $p_{inlet}^j$ est la pression dans le premier nœud du premier compartiment de $\Omega_M^j$.

**8.** - Procédé mis en œuvre par ordinateur selon une ou plusieurs des revendications précédentes, **caractérisé par le fait que** ladite étape de modification (33) comprend la multiplication des coefficients de couplage de pression inter-compartiments ($\beta_{1,2}$, $\beta_{2,3}$) par un facteur prédéfini dans les régions de perfusion septales.

**9.** - Appareil d'angiographie coronarienne par tomographie assistée par ordinateur au repos (cCTA) configuré pour exécuter les étapes du procédé mis en œuvre par ordinateur (1) pour la simulation d'un débit sanguin myocardique dans des conditions de stress selon une ou plusieurs des revendications précédentes.

Fig.1

REST CONDITIONS

CENTERLINES

STRESS CONDITIONS

CENTERLINES
COMPUTATION

TUTBULAR SURFACES

s

$r_{rest}$

$u_{str}r_{rest}$

A

B

C

Fig.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2021334963 A1

### Non-patent literature cited in the description

- A computational model applied to myocardial perfusion in the human heart: From large coronaries to microvasculature. *Journal of Computational Physics*, 2021, vol. 424, 109836
- The multi-scale modelling of coronary blood flow. *Annals of Biomedical Engineering*, 2012, vol. 40 (11), 2399-2413
- Multi-scale parameterisation of a myocardial perfusion model using whole-organ arterial networks. *Annals of Biomedical Engineering*, 2014, vol. 42 (4), 797-811
- *Medical and Biological Engineering and Computing*, 2005, vol. 43 (4), 431-435